# EUROPEAN PATENT APPLICATION

(11) **EP 4 775 193 A1**
(43) Date of publication of application: **15.07.2026**
(21) Application number: 25206543.8
(22) Date of filing: 03.10.2025
(51) Int. Cl.: A61H 33/06, G05B 15/02

(54) **SYSTEM FOR PROVIDING AN INDIVIDUALIZED ENVIRONMENT FOR REST, WELLNESS AND SPORTS**

(71) Applicant: Aizelman, Alexandr, 1077 Budapest (HU)
(72) Inventor: Aizelman, Alexandr, 1077 Budapest (HU)

(57) **Abstract**

A system for providing an individualized environment for rest, wellness and sports consists of at least one functional module (1) for providing a given physical effect in the room or zone for rest and/or wellness and/or sports by means of a device (2) for providing the given physical effect, and a control module (4). The functional module is equipped with a control device (3), at least one input connector for connecting to engineering networks, and at least one output connector for supplying the given physical effect to the room or zone for rest and/or wellness and/or sports. The system allows promptly enabling and disabling the modules without changing the stationary engineering networks, performing local adjustment of the functional modules, and ensuring operation of the functional modules without constant interaction with the control module.

## Description

### Field of the invention

The present invention relates to equipment for areas intended for rest and/or wellness and/or sports, in particular to systems configured to create climatic, lighting, acoustic, aromatic and other effects that facilitate formation of a specified individualized psycho-emotional environment (e.g., relaxing, toning or the like). The invention may be attributed to the technical field of equipment for dedicated areas and enclosed spaces, including devices that provide a combined action of temperature, humidity, light, sound and aromas on the human body to improve well-being and to produce a desired emotional and physiological response.

### Background of the invention

The prior art teaches various technical solutions concerning providing an adjustable atmosphere, in particular systems that provide climatic and atmospheric effects, including temperature and humidity adjustment, aroma generation, and providing illuminative, visual, and acoustic effects. Typically, the known systems are characterized by automated management, the possibility of parameter personalization, and remote control.

In particular, a sauna comprising system for generation of scented smoke, sound of fire and light effects is known (Sauna, comprising devices for generation of scented smoke, sound of fire, and light effects, 20216898, DE, A61H33/06, Application number 20216898, DE, 02.11.2002, published 15.04.2004). The system comprises an artificial smoke or smoky scented agent generator, a ventilation device, light effects devices (including colored illumination), as well as background sound. The management is performed by means of a microprocessor according to preset programs, thereby synchronizing operation of ventilation, smoke generation, illumination and sound effects. However, the equipment is designed as built-in communication lines, so it is not possible to introduce changes into a set of effects or a system configuration without performing a complex rearrangement of the entire room.

An integrated sauna management system is known (Sauna system with communication, networking, and global infrastructure, 11491079, US, ZACK A.M. et al., Application number US201916549432A, 23.08.2019, A61H33/00; A61H33/06; G06F15/16; H04L67/12; published 08.11.2022), the system comprises a video display, an acoustic system, aromatherapy means, lighting, as well as interfaces for personalized user interaction. The system implies centralized management via remote servers to allow storage of user profiles and automation of scenarios depending on user preferences. However, this system is not built into individual structural elements of the sauna and does not allow flexible configuration.

Besides, a system for air bath room is known (Air bathing rest room, Publication 2004181033, JP, UCHIBORI Yoshitaka et al., A61H33/06; A61M21/02; F24F11/02; Application number JP2002353256A, 05.12.2002, published 02.07.2004), where the atmosphere is adjusted according to preset programs, including parameters of time, temperature, humidity, illumination, and sound effects. The system implies aroma generation, provision of air flows of various intensities, the possibility of projecting visual effects, as well as sounds and music playback. The known solution is aimed at providing a comfortable and relaxing atmosphere in a specifically designed room with integrated utility systems. Therewith, the structure of the system also does not allow any flexible configuration or expansion, since all devices are initially integrated into individual structural elements of the room.

However, when using these systems in practice, it is often necessary to vary or update a set of effects being used, e.g., depending on needs of visitors, season change, new developed programs or during integration of additional equipment. For such purposes, it is important to have a technical solution that allows changing the system configuration for flexible expansion or modification of the set of effects, without carrying out major construction works.

Therefore, the prior art demonstrates a trend toward systems designed to create an individualized environment for rest, wellness, and sports; however, there is no solution that provides a modular architecture with autonomous units enabling flexible integration and expansion of system functionality without the need for complex installation or reconstruction of rest, wellness, or sports areas.

The objective of the present invention is to develop a modular system for providing an individualized environment for rest and/or wellness and/or sports that could ensure the provision of physical effects by means of special autonomous modules.

### Summary of the invention

The objective is achieved by providing a system for providing an individualized environment for rest, wellness and sports, comprising at least one functional unit for providing of a given physical effect in the room or zone for rest and/or wellness and/or sports that is equipped with at least one device for providing the given physical effect, and a control unit for controlling the functional unit, wherein each unit is made as a separate module arranged in a housing, the functional module comprises a control device, is equipped with at least one input connector for connecting to engineering networks, and at least one output connector connected to the device for providing the given physical effect and configured to supply the given physical effect to the room or zone for rest and/or wellness and/or sports.

Besides, the functional module is configured to generate a multimedia content or audio content, or to generate aroma, or to form light effects, or to form air flows, or to form water effects.

Besides, the control module comprises an audio signal generation device and a line-level audio output for connection to acoustic devices.

Besides, the input connectors comprise connectors for connection to a power-supply engineering network.

Besides, the the input connectors comprise connectors for connection to water-supply engineering networks.

Besides, the input connectors comprise connectors for connection to ventilation engineering networks.

Besides, the output connectors comprise connectors for connecting to an external device for supply of water or atomized water or vapor or fog or air, or cable connectors for connecting to external illumination devices or acoustic devices or multimedia devices mounted in the room or zone for rest and/or wellness and/or sports.

Besides, the functional modules are made interconnectable.

Besides, the control module is configured to remotely control the functional modules.

Besides, the control module is configured to remotely interact with external control user devices or systems.

The engineering networks are selected from the group consisting of an electrical network, a water supply network, a gas supply network, a heat supply network, a sewerage network, lighting, and other facility equipment.

Owing to making each of the functional units as an autonomous module in a separate housing that is equipped with at least one input connector for connecting to engineering networks and at least one output connector for supplying the provided physical, including, inter alia, light, audio, or video effect to the room or zone for rest and/or wellness and/or sports, it is possible to promptly enable and disable these modules without changing the stationary engineering networks. Owing to equipping the functional module with its own control device, it is possible to perform its local adjustment, including individual programs with operation parameters, as well as to ensure operation of the module as a part of the system without any constant interaction with the control module, thereby enhancing system reliability.

Owing to making the input connectors of the modules with connectors for connecting to power supply, water supply, or ventilation engineering networks, convenient installation and maintenance are ensured, as well as the dismounting and replacement of individual modules. This also enables flexible modification and expansion of the system without altering building structures or installing new stationary engineering networks in the room or in a zone for rest and/or wellness and/or sports.

The interconnectability of the modules allows forming both autonomous and integrated complexes with flexible scalability of the system depending on the desired effects.

Owing to the fact that the control module is configured to remotely control the functional modules, usability is increased. Owing to the possibility of remote interaction between the control module and external user devices or systems (e.g., smartphones, tablets, smart home systems, automated control systems), integration of the claimed system into modern intelligent infrastructures is ensured, thereby further expanding its functional and operational possibilities.

### Brief description of the drawings

These and other features of the invention will be explained by describing the best embodiment of the invention that is stated herein below and presented as a non-limiting example, with a reference to accompanying drawings, where:
Fig. 1 illustrates a functional diagram of the modular system for providing an individualized environment for rest, wellness and sports
Fig. 2 illustrates a variant of the functional diagram of the modular system for providing an individualized environment for rest, wellness and sports.

### Description of exemplary embodiment of the invention

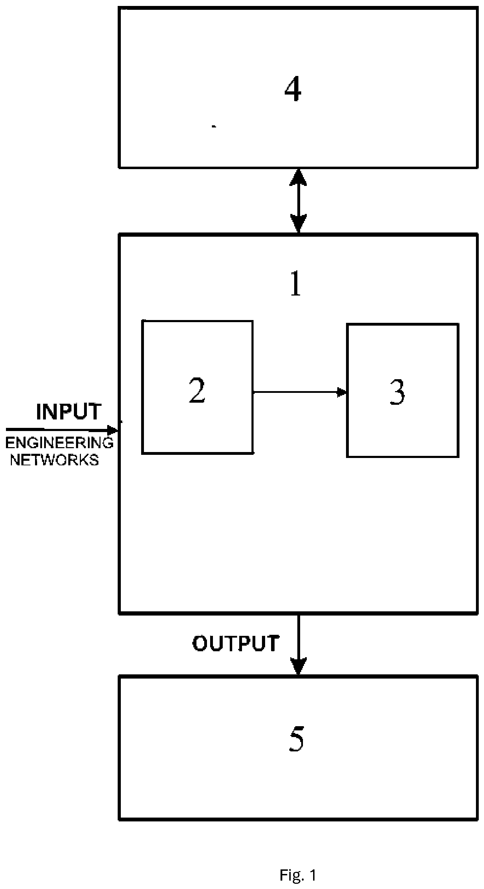

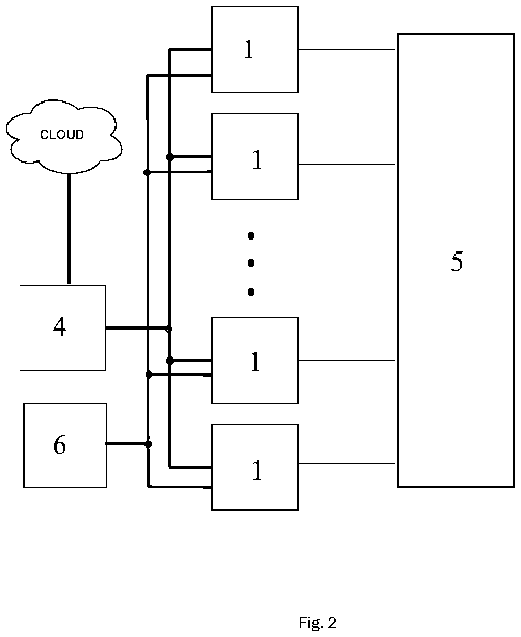

The system consists of at least one functional module (1) and a control module (4) (Fig. 1). The functional module is responsible for providing a certain physical effect in the room or zone for rest and/or wellness and/or sports by means of a device (2) for providing the given physical effect therein. The functional module is equipped with a control device (3) (Fig. 1), at least one input connector for connecting to engineering networks (6), and at least one output connector for supplying the provided effect to the room or zone for rest and/or wellness and/or sports (5) (Fig. 2). The output connector may be, e.g., pipelines for supplying water, vapor, or fog (for example, to ceiling or wall nozzles); air ducts for supplying air to diffusers or ventilation grids; or cable connectors for supplying signals to external lamps or acoustic devices mounted in the room or zone for rest and/or wellness and/or sports. The control device (3) within the module controls the operation of the device (2) for providing the effect and the entire auxiliary equipment of the module. The control module (4) performs centralized management: it receives commands from a user (or an external automation system) and either enables/disables the modules or adjusts their operation according to given parameters. The system is designed according to the modular principle: the functional module (1) is made as a separate module in an individual housing. At the same time, owing to the presence of the individual control device (3) in each of the functional modules (1), they can operate independently of each other, thereby allowing each module to be set and used separately as an autonomous device.

This means that the effects may be added or replaced by simply enabling or disabling the corresponding functional modules, e.g., to set only the necessary functional modules for the room or zone for rest and/or wellness and/or sports or to replace one functional module with another when performing modernization (Fig. 2).

The system for providing an individualized environment for rest and/or wellness and/or sports may comprise, e.g., the following functional modules.

A fog/vapor generation module ("FOG") comprises a device for providing a water fog or vapor effect (e.g., an ultrasonic fog generator) and a fan for distributing the provided vapor/fog in the room. This module is connected to a water supply system (for supplying water to the generator) and to a power supply network (for supplying power to the generator, fan, and controller). A local controller controls the fog generator and the fan, thereby ensuring enabling/disabling and setting the required duration/intensity of the fog effect. In this case, the output connector is a nozzle or an opening through which the vapor or fog is supplied to the room or zone for rest and/or wellness and/or sports.

A light effects module ("LIGHT") is intended for providing chromotherapy and illuminating the room or zone for rest and/or wellness and/or sports. The corresponding lighting drivers/controllers are arranged in the housing of this module. The LIGHT module is mainly connected to the power supply, and its local control unit may provide various lighting scenarios (color change, intensity adjustment, flashing, and other light effects). Output connectors for connecting lamps or light strips mounted in the room or zone for rest and/or wellness and/or sports may be arranged on an outer panel of the module.

A "rain" module ("RAIN") provides a shower effect, e.g., a torrential rain effect. The structure of this module usually comprises a pump, one or more electromagnetic valves for controlling water supply, as well as a heating element for warm water supply. The RAIN module is connected, via input connectors, to the water supply system (cold water supplied to the input), to the power supply network (for supplying power to the pump, heater, and control system), and, if necessary, to a water drainage system (for discharging excessive water). A local controller of the module controls, e.g., an operation sequence of the devices comprised in the module. In this case, the output connectors are outputs for connecting to the water supply pipelines, e.g., to ceiling or wall nozzles mounted on a ceiling or walls of the room or zone for rest and/or wellness and/or sports, through which the rain water is supplied.

An air aromatization module ("AROMA") provides an aromatherapy effect by distributing aromatic substances. The module may comprise one or more reservoirs with aromatic liquid or essential oils and dispensing devices, e.g., a compressor with a nozzle that converts the liquid into fog. The AROMA module is connected to the power supply network for supplying power to the devices within the module; if necessary, it may also be connected to a compressed air system. A local control unit adjusts the periodicity and duration of dispensing and may control switching between different aromas. The output connector is an output for connecting to a pipeline, through which the aromatized air is supplied.

An air effects module ("AIR") is intended to provide air flows, ventilation, or imitation of a light breeze. Usually, this module comprises at least one fan for supplying fresh air and/or an exhaust fan for discharging air (both fans may be combined in a single module in order to ensure circulation). The AIR module is connected, via input connectors, to a power supply system, and it may also be connected to the ventilation channels of a building (e.g., via sockets for intaking fresh air from outside and discharging exhaust air to the ventilation system). An internal controller of the module controls the operation of the fans, e.g., changes their rotation speed or alternately enables them in order to achieve the desired effect (cold air inflow, aeration between vapor emission cycles, and the like). The provided air effect is output to an air duct that extends to grids or diffusers, which output the air flow (cooled down or fresh air).

A multimedia module is intended to provide audiovisual effects. For example, this module may comprise a multimedia content playback device such as a projector or a display for visual effects, or an audio system for background music. In the simplest case, the background music is provided by the control module via acoustic devices connected thereto.

All the listed modules share the following principle: each of them has its own intelligent control and is capable of operating independently of other modules. This significantly simplifies the integration of the system: it is easy to add, combine, or replace the functional units without introducing any changes into the electronics of other modules. For example, a light effects module may be additionally installed in an already existing system, and it will perform the programmed lighting scenarios. Similarly, if any of the modules is temporarily unnecessary, it may be disabled or removed without interrupting the operation of the remaining system components.

In order to control and coordinate the operation of all the functional units, the system comprises the control module (4) (see Fig. 2). The control module (4) communicates with each of the functional modules (1), and this communication may be either wired (e.g., via a shared bus or through the power supply line) or wireless. In a particular embodiment, a wireless communication transceiver, e.g., a radio module of a certain range, is embedded in the structure of each of the functional modules, thereby allowing the control module to exchange commands with the modules in a wireless manner. The control module (4) acts as a main controller of the system: the user or an external system may control all the effects via the same in a comprehensive manner. For example, the control module (4) allows synchronizing the operation of various functional modules (1) for simultaneous provision of a complex physical effect (light, sound, aroma, and vapor may be enabled synchronously according to a scenario). Also, scenarios or modes (e.g., "a tropical forest mode" includes aromatization and jungle sounds in combination with fog and dimmed green light) may be set via the single controller.

The control module (4) may comprise an audio signal generation device (a multimedia player or a sound generator) and a corresponding audio output, e.g., a line-level audio output for connecting to external acoustic systems (speakers). Therefore, the control module is capable of providing an audio background for the atmospheric effects being provided, such as music, nature sounds, or voice instructions that supplement the provided atmosphere.

The control module (4) is also intended for remote control and external communication. The control module may be connected to a network (Fig. 2), for example, to the Internet via a cloud service, for remote monitoring and control of the system. In addition, it is capable of interacting with external user devices, e.g., a smartphone, a tablet, or a stationary controller. This allows the user to activate the required functional modules (1) remotely, to change scenario settings, to track the status of the system via a mobile application, or to integrate systems control into a smart home system. The connection to the external devices may be performed either via wireless technologies (Wi-Fi, Bluetooth, radio channel, etc.) or via a wired interface. Therefore, the possibilities of remote control of the functional modules and interaction with external systems are provided.

In a regular usage mode, the user interacts with the system via the control module (or an external device connected thereto) by transmitting commands for activation of the required effects or scenario selection; the control module processes these commands and distributes them across the functional modules. The presence of intelligent local control devices in the functional modules reduces the load of the control module and increases reliability. For example, a local control device may independently maintain the required parameters within the functional module, such as water temperature in the "RAIN" module or lighting intensity in the "LIGHT" module, without any continuous requests to the control module, thereby accelerating the system response and reducing the load of the control module.

Besides, in case of faults or temporary communication failures, the control device of each of the functional modules can ensure a safe state of its equipment (e.g., disabling the heater in case of overheating or stopping the pump if there is no control signal) and inform the control module about the problem after communication is restored. At the same time, other system components continue operating, and the control module, if necessary, may correct the scenario while compensating for the absence of a physical effect or timely informing the user about the fault.

Therefore, the modular building principle of the system is combined with centralized control: each of the functional units is designed as an autonomous functional module with a control device in order to provide its own effect, while coordination and combined control of all the functional modules are performed by the control module. This architecture allows providing an individualized environment in the room or zone for rest and/or wellness and/or sports by easily adapting the set and the range of effects to the user's preferences. At the same time, high usability and adjustment flexibility are achieved due to the distributed control system that combines the local control of individual components with the advantages of centralized control.

## Claims

1. System for providing an individualized environment for rest, wellness and sports, comprising at least one functional unit for providing of a given physical effect in the room or zone for rest and/or wellness and/or sports that is equipped with at least one device for providing the given physical effect, and a control unit for controlling the functional unit, **characterised in that** each unit is made as a separate module arranged in a housing, the functional module comprises a control device, is equipped with at least one input connector for connecting to engineering networks, and at least one output connector connected to the device for providing the given physical effect and configured to supply the given physical effect to the room or zone for rest and/or wellness and/or sports.

2. System according to claim 1, **characterised in that** the functional module is configured to generate a multimedia content or audio content, or to generate aroma, or to form light effects, or to form airflows, or to form water effects.

3. System according to claim 1, **characterised in that** the control module comprises an audio signal generation device and a line-level audio output for connection to acoustic devices.

4. System according to claim 1 or 2, **characterised in that** the input connectors comprise connectors for connection to a power-supply engineering network.

5. System according to claim 1 or 2, **characterised in that** the input connectors comprise connectors for connection to water-supply engineering networks.

6. System according to claim 1 or 2, **characterised in that** the input connectors comprise connectors for connection to ventilation engineering networks.

7. System according to any one of claims 1-6, **characterised in that** the output connectors comprise connectors for connecting to an external device for supply of water or atomized water or vapor or fog or air, or cable connectors for connecting to external lighting devices or acoustic devices or multimedia devices mounted in the room or zone for rest and/or wellness and/or sports.

8. System according to any one of claims 1-7, **characterised in that** the functional modules are made interconnectable.

9. System according to any one of claims 1-8, **characterised in that** the control module is configured to remotely control the functional modules.

10. System according to any one of claims 1-9, **characterised in that** the control module is configured to remotely interact with external control user devices or systems.

## Amended claims

### Amended claims in accordance with Rule 137(2) EPC.

1. System for providing an individualized environment for rest, wellness and sports, comprising a control unit, as well as a functional unit for generating multimedia content or audio content, arranged in a separate housing, equipped with at least one input connector for connection to engineering networks and at least one output connector for supplying multimedia content or audio content to a room or zone for rest and/or wellness and/or sports, said functional unit being equipped with an intelligent control device, and at least one functional unit for providing given physical effects in a room or zone for rest and/or wellness and/or sports, other than multimedia effects or audio content, equipped with at least one device for creating the given physical effect, **characterised in that** the functional unit for providing given physical effects and the control unit are arranged in separate housings, wherein the functional unit for providing given physical effects is equipped with an intelligent control device configured to control the device for creating the corresponding physical effect and to locally adjust the parameters of the functional unit, as well as with at least one input connector for connection to engineering networks and at least one output connector connected to the device for creating the given physical effect and configured to supply the working medium of the given physical effect to the room or zone for rest and/or wellness and/or sports, and the control unit is configured to set operation scenarios for the functional units and to perform synchronized control of at least two functional units in accordance with the set scenario.

2. System according to claim 1, **characterised in that** the functional unit for providing given physical effects is configured to generate aroma, or to form light effects, or to form airflows, or to form water effects.

3. System according to claim 1, **characterised in that** the control unit comprises an audio signal generation device and a line-level audio output for connection to acoustic devices.

4. System according to claim 1 or 2, **characterised in that** the input connectors comprise connectors for connection to a power-supply engineering network.

5. System according to claim 1 or 2, **characterised in that** the input connectors comprise connectors for connection to water-supply engineering networks.

6. System according to claim 1 or 2, **characterised in that** the input connectors comprise connectors for connection to ventilation engineering networks.

7. System according to any one of claims 1-6, **characterised in that** the output connectors comprise connectors for connecting to an external device for supply of water or atomized water or vapor or fog or air, or cable connectors for connecting to external lighting devices or acoustic devices or multimedia devices mounted in the room or zone for rest and/or wellness and/or sports.

8. System according to any one of claims 1-7, **characterised in that** the functional units are made interconnectable.

9. System according to any one of claims 1-8, **characterised in that** the control unit is configured to remotely control the functional units.

10. System according to any one of claims 1-9, **characterised in that** the control unit is configured to remotely interact with external control user devices or systems.
